Europäisches Patentamt

European Patent Office

Office européen des brevets

(19)

(11) Veröffentlichungsnummer : **0 149 422**
**B1**

(12) **EUROPÄISCHE PATENTSCHRIFT**

(45) Veröffentlichungstag der Patentschrift :
17.05.89

(21) Anmeldenummer : 84810587.0

(22) Anmeldetag : 03.12.84

(51) Int. Cl.⁴ : **C 07 C 85/24, C 07 C 87/54,
C 09 K 15/18, C 10 M133/12**

(54) Antioxidans-Herstellung.

(30) Priorität : 08.12.83 GB 8332797

(43) Veröffentlichungstag der Anmeldung :
24.07.85 Patentblatt 85/30

(45) Bekanntmachung des Hinweises auf die Patenterteilung : 17.05.89 Patentblatt 89/20

(84) Benannte Vertragsstaaten :
**BE DE FR GB IT**

(56) Entgegenhaltungen :
**DE-A- 2 111 194
GB-A- 846 226
GB-A- 1 143 250
GB-A- 2 005 260**

(73) Patentinhaber : **CIBA-GEIGY AG
Klybeckstrasse 141
CH-4002 Basel (CH)**

(72) Erfinder : **Franklin, Janet
100 Shelfield Lane, Norden
Rochdale, Lancashire OL11 5XZ (GB)**

Jouve, 18, rue St-Denis, 75001 Paris, France

EP 0 149 422 B1

## EP 0 149 422 B1

**Beschreibung**

Die vorliegende Erfindung betrifft ein Verfahren zur Herstellung einer flüssigen Antioxidans-Zusammensetzung, das so hergestellte Produkt und seine Verwendung als Stabilisator von organischem Material.

Es ist ein Verfahren bekannt, gemäss dem eine dunkelgefärbte Zusammensetzung durch Reaktion von Diphenylamin mit Diisobutylen bei Anwesenheit von Aluminiumchlorid als Katalysator hergestellt wird. Das so erhaltene Produkt weist einen Gehalt von ungefähr 90 Gew.-% an Di-tert.-octyldiphenylamin und ca. 10 Gew.-% an Mono-tert.-octyl- und gemischten Tert.-butyl-tert.-octyl-Diphenylaminen auf.

Solche festen Antioxidans-Zusammensetzungen sind unvorteilhaft in Bezug auf den Umgang damit und den Transport sowie auf die Leichtigkeit der Einarbeitung in das zu stabilisierende Substrat.

Flüssige Antioxidans-Zusammensetzungen, welche einfach gehandhabt und leicht eingearbeitet werden können und sich von Diphenylamin und Olefinen herleiten, sind bekannt und z. B. in GB-A 1 332 201 erwähnt.

Die GB-A 1 143 250 beschreibt beispielsweise die Herstellung von festen Dioctyl- oder -nonyldiphenylaminen durch Alkylierung von Diphenylamin mit einem entsprechenden Olefin in Gegenwart eines Friedel-Crafts-Katalysators.

Die DE-A 2 111 194 erwähnt die Alkylierung von Aminen mit Olefinen, wobei ein Tonerde-Katalysator, dessen Wirksamkeit ausschließlich auf schwach säuren Funktionen beruht, zur Anwendung kommt.

Es wurde nun gefunden, dass sich durch Reaktion von Diphenylamin mit Diisobutylen unter spezifischen Reaktionsbedingungen eine flüssige Antioxidans-Zusammensetzung mit hervorragenden Antioxidans-Eigenschaften herstellen lässt.

Die vorliegende Erfindung betrifft ein Verfahren zur Herstellung einer flüssigen Antioxidans-Zusammensetzung, dadurch gekennzeichnet, dass die Reaktion von Diphenylamin mit einem Ueberschuss an Diisobutylen in Anwesenheit eines aktiven Tonerde-Katalysators durchgeführt wird, dass die Konzentration an Diisobutylen über die Reaktionsdauer im Wesentlichen konstant gehalten wird, dass die Reaktionstemperatur mindestens 160 °C beträgt, dass die Reaktion solange durchgeführt wird bis der Gehalt an 4,4'-Di-tert.-octyldiphenylamin, bezogen auf die Reaktionsmasse ohne Katalysator, unter 30 Gew.-%, vorzugsweise unter 25 Gew.-% und der Gehalt an Diphenylamin unter 10 Gew.-%, vorzugsweise unter 5 Gew.-% liegen, dass der Katalysator und nicht umgesetztes Diisobutylen entfernt werden und dass das entstehende flüssige Produkt, enthaltend mindestens die Komponenten 1) tert.-Butyldiphenylamine, 2) tert.-Octyldiphenylamine und 3) höher alkylierte Diphenylamine isoliert wird.

Der Begriff « tert.-Octyl » bedeutet in der Offenbarung und in den Ansprüchen hiernach 2,2,4,-Trimethyl-4-pentyl.

Die Reaktion kann zweckmässig durch Einfüllen des Diphenylamins und des Katalysators in das Reaktionsgefäss und durch Aufheizen der Mischung auf mindestens 160 °C, vorzugsweise auf mindestens 165 °C und vorzugsweise unter Rühren durchgeführt werden. Darauf wird Diisobutylen so zum heissen Gemisch von Diphenylamin und Katalysator zudosiert, dass die Temperatur des Gemischs nicht unter 160 °C, vorzugsweise nicht unter 165 °C absinkt.

Unter Wärmezufuhr und Rühren wird die Temperatur auf mindestens 160 °C gehalten unter häufiger Probenahme bis das Produkt ohne Katalysator, weniger als 30 Gew.-% an 4,4'-Di-tert.-octyldiphenylamin und weniger als 10 Gew.-% an Diphenylamin enthält.

Die Temperatur, bei der das erfindungsgemässe Verfahren durchgeführt wird, beträgt mindestens 160 °C, kann aber beträchtlich höher sein z. B. bis 250 °C.

Um das Abbaurisiko zu vermindern liegt das übliche Temperaturmaximum ungefähr bei 190 °C.

Die Zeit, über die das Diisobutylen zum heissen Gemisch von Diphenylamin und Katalysator zugegeben werden kann, kann abhängig von der Reaktionstemperatur in einem grossen Bereich schwanken, liegt aber üblicherweise innerhalb von 3-30 Stunden.

Das Molverhältnis von Diphenylamin zu Diisobutylen kann über einen weiten Bereich variieren, wird aber bevorzugt im Bereich von 1 : 1.11 bis 1 : 2.5, besonders bevorzugt 1 : 1,3 bis 1 : 1,75 gehalten, um die Kosten für Ausgangsmaterial zu vermindern und um die Zugabezeit von Diisobutylen möglichst klein zu halten.

Die Rückgewinnung des Katalysators erfolgt zweckmässigerweise durch Vakuumfiltration des heissen Reaktionsgemischs. Die Rückgewinnung von überschüssigem Diisobutylen kann leicht durch Vakuumdestillation des Reaktionsgemischs erfolgen.

Der im erfindungsgemässen Verfahren verwendete aktive Tonerde-Katalysator weist vorzugsweise einen freien Feuchtigkeitsgehalt von unter 10 Gew.%, besonders bevorzugt einen solchen von unter 5 Gew.-% auf.

Im Handel erhältliche Katalysatoren, welche sich als wirksam erweisen, sind z. B. Fulcat[®] 14, Fulmont[®] 700C, Fulmont[®] 237, Katalysator K-10 (Süd-Chemie) und bevorzugt Fulcat[®] 22B (eine mit Schwefelsäure aktivierte Tonerde). Die Fulcat und Fulmont Katalysatoren sind im Handel von Laporte Industries erhältlich.

Die im erfindungsgemässen Verfahren verwendeten aktiven Tonerde-Katalysatoren zeigen gegenüber den bekannten Aluminiumchlorid-Katalysatoren folgende Ueberlegenheit :

2

a) Katalysator-Abscheidung kann durch gewöhnliche Filtration erfolgen und

b) aktive Tonerden, welche Bleicheigenschaften aufweisen, ergeben viel heller gefärbte Endprodukte als solche, die unter Verwendung von Aluminiumchlorid-Katalysatoren erhalten werden.

Die im erfindungsgemässen Verfahren verwendete Katalysatormenge kann in einem breiten Bereich schwanken ohne negative Auswirkung auf den Reaktionsablauf. In der praktischen Anwendung wird der Katalysator vorzugsweise in einer Menge von 5-25 Gew.%, insbesondere bevorzugt von 5-15 Gew.% bezogen auf das Gewicht der Diphenylamin-Reaktionskomponente eingesetzt.

Die nach dem erfindungsgemässen Verfahren hergestellten Produkte enthalten mindestens die folgenden Komponenten.

1) tert.-Butyldiphenylamine,
2) tert.-Octyldiphenylamine und
3) höher alkylierte Diphenylamine.

Die nach dem erfindungsgemässen Verfahren hergestellten flüssigen Antioxidantien können als Antioxidantien in einem weiten Bereich von organischem Material eingesetzt werden.

Die vorliegende Erfindung betrifft im weitern eine Zusammensetzung enthaltend ein dem oxidativen Abbau unterliegendes organisches Material und als Stabilisator, einen stabilisierenden Anteil eines nach dem erfindungsgemässen Verfahren hergestellten flüssigen Antioxidans.

Eine besondere Klasse von dem oxidativen Abbau unterliegendem organischem Material, für die die nach dem erfindungsgemässen Verfahren hergestellten Produkte wertvolle Stabilisatoren darstellen, sind Schmiermittel und funktionelle Flüssigkeiten auf Mineralöl-Basis oder synthetische Schmiermittel oder funktionelle Flüssigkeiten, insbesondere die, welche Carbonsäure-Esterderivate darstellen und bei Temperaturen von 200 ºC und höher verwendet werden.

Beispiele von synthetischen Schmiermitteln umfassen Schmiermittel auf der Basis eines Diesters einer zweiwertigen Säure mit einem einwertigen Alkohol wie z. B. Dioctylsebacat oder Dinonyladipat ; eines Triesters von Trimethylolpropan mit einer einwertigen Säure oder mit einem Gemisch solcher Säuren wie z. B. Trimethylolpropantripelargonat, Trimethylolpropan-tricaprylat oder Gemische davon ; eines Tetraesters von Pentaerythrit mit einer einwertigen Säure oder mit einem Gemisch solcher Säuren wie z. B. Pentaerythrit-tetracaprylat : oder eines komplexen Esters von einwertigen und zweiwertigen Säuren mit mehrwertigen Alkoholen wie z. B. ein komplexer Ester von Trimethylopropan mit Capryl- und Sebacinsäure oder von einem Gemisch davon.

Weitere synthetische Schmiermittel sind dem Fachmann geläufig und z. B. im « Schmiermittel-Taschenbuch » (Hüthig Verlag, Heidelberg 1974) beschrieben.

Besonders geeignet sind neben den bevorzugten Mineralölen z. B. Phosphate, Glykole, Polyglykole, Polyalkylenglykole und Poly-α-Olefine.

Weiteres organisches, dem oxidativen Abbau unterliegendes Material, für das die nach dem erfindungsgemässen Verfahren hergestellten Produkte besonders wertvolle Stabilisatoren darstellen, schliesst Substanzen aus den folgenden Klassen ein :

a) Natürliche und synthetische Polymere wie z. B. Naturkautschuk ; synthetische Additionspolymere wie Homo- und Copolymere von Vinyl- und Vinyliden-Monomeren, einschliesslich Ethylen, Propylen, Styrol, Butadien, Isopren, Acrylnitril, Vinylchlorid oder Vinylacetat ; synthetische Polymere aus Kondensationsreaktionen enthaltend Aether-, Ester- (Carbonsäure-, Schwefelsäure- und Kohlensäureester), Amid- oder Urethangruppierungen wie z. B. Alkyd- und Polyamid-harze, wobei im Falle dieser Polymere das flüssige Antioxidans während einer Polymer-Verarbeitungsstufe wie z. B. einem Mischen von Kautschuk zugesetzt werden kann.

b) Nicht polymere sauerstoffhaltige Verbindungen, wie z. B. Aldehyde wie n-Heptaldehyd und ungesättigte Fettsäuren oder Fettsäureester wie Methyloleat oder Ricinussäure.

In Bezug auf dieses vorstehend erwähnte weitere organische Material sind die nach dem erfindungsgemässen Verfahren hergestellten Antioxidantien wirksame Stabilisatoren für Kautschuke. Sie können in Natur- und synthetischen Kautschuken eingesetzt werden. Ein Beispiel für synthetischen Kautschuk ist SBR (Styrol-Butadien-Kautschuk).

Andererseits können die Verfahrensprodukte als Stabilisatoren für Gemische aus Natur- und synthetischen Kautschuk eingesetzt werden wie z. B. für eine Mischung aus Natur- und Styrol-Butadien-Kautschuk.

Die nach dem erfindungsgemässen Verfahren hergestellen Antioxidantien können auch in Mehrstoff-Zusammensetzungen verwendet werden, das sind Zusammensetzungen enthaltend mindestens eine dem oxidativen Abbau unterliegende Verbindung oder eines Gemisches davon und eine oder mehrere organische oder anorganische Verbindungen z. B. eine alkoholische oder wässrige Emulsion von

3

organischem dem oxidativen Abbau unterliegenden Material.

Die erfindungsgemässen Zusammensetzungen enthalten vorzugsweise einen Anteil an nach dem erfindungsgemässen Verfahren hergestellten flüssigen Antioxidans im Bereich von 0.05-5.0 Gew.-% bezogen auf das Gewicht des organischen Materials. Besonders bevorzugt enthalten die Zusammensetzungen einen Anteil von 0.1-4.0 Gew.% an flüssigem Antioxidans bezogen auf das Gewicht des organischen Materials.

Die Menge an eingesetztem Antioxidans in einem bestimmten organischen Material hängt nicht nur von der Art des organischen Materials ab sondern auch von den äusseren Bedingungen, unter welchen das organische Material verwendet wird. So enthält organisches Material, das bei normalen Temperaturen verwendet wird, normalerweise einen kleineren Anteil an flüssigem Antioxidans als solches, wie z. B. synthetische Schmiermittel, das zur Verwendung bei erhöhten Temperaturen vorgesehen ist.

Zusätzlich zu dem flüssigen Antioxidans können die Schmiermittel andere Additive enthalten, die zugegeben werden, um die Grundeigenschaften von Schmiermitteln noch weiter zu verbessern ; dazu gehören : Andere Antioxidantien, Metallpassivatoren, Rostinhibitoren, Viskositätsindex-Verbesserer, Stockpunkterniedriger, Dispergiermittel, Detergentien und Antiverschleiss-Additive.

Beispiele für phenolische Antioxidantien

1.1. Alkylierte Monophenole

2,6-Di-tert.butyl-4-methylphenol
2,6-Di-tert.-butylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

1.2. Alkylierte Hydrochinone

2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methylen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]

Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5. Benzylverbindungen

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

1.6. Acylaminophenole

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure mit ein- oder mehrwertigen Alkoholen, wie z. B. mit

Methanol                                    Diethylenglycol
Octadecanol                                 Triethylenglycol
1,6-Hexandiol                               Pentaerythrit
Neopentylglycol                             Tris-hydroxyethyl-isocyanurat
Thiodiethylenglycol                         Di-hydroxyethyl-oxalsäurediamid

1.8. Ester der β-(5-tert.butyl-4-hydroxy-3-methylphenyl)-propionsäure mit ein- oder mehrwertigen Alkoholen, wie z. B. mit

Methanol                                    Diethylenglycol
Octadecanol                                 Triethylenglycol
1,6-Hexandiol                               Pentaerythrit
Neopentylglycol                             Tris-hydroxyethyl-isocyanurat
Thiodiethylenglycol                         Di-hydroxyethyl-oxalsäurediamid

1.9. Amide der β-(3,5-Di-tert.butyl-4-hydroxyphenyl)-propionsäure, wie z. B.

N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

Beispiele für aminische Antioxidantien

N,N'-Di-isopropyl-p-phenylendiamin
N,N'-Di-sec-butyl-p-phenylendiamin
N,N'-Bis(1,4-dimethyl-pentyl)-p-phenylendiamin
N,N'-Bis(1-ethyl-3-methyl-pentyl)-p-phenylendiamin
N,N'-Bis(1-methyl-heptyl)-p-phenylendiamin
N,N'-Diphenyl-p-phenylendiamin
N,N'-Di-(naphthyl-2-)-p-phenylendiamin
N-Isopropyl-N'-phenyl-p-phenylendiamin
N-(1,3-Dimethyl-butyl)-N'-phenyl-p-phenylendiamin
N-(1-Methyl-heptyl)-N'-phenyl-p-phenylendiamin
N-Cyclohexyl-N'-phenyl-p-phenylendiamin
4-(p-Toluol-sulfonamido)-diphenylamin
N,N'-Dimethyl-N,N'-di-sec-butyl-p-phenylendiamin
Diphenylamin
4-Isopropoxy-diphenylamin
N-Phenyl-1-naphthylamin
N-Phenyl-2-naphthylamin
octyliertes Diphenylamin

4-n-Butylaminophenol
4-Butyrylamino-phenol
4-Nonanoylamino-phenol
4-Dodecanoylamino-phenol
4-Octadecanoylamino-phenol
Di-(4-methoxy-phenyl)-amin
2,6-Di-tert.butyl-4-dimethylamino-methyl-phenol
2,4'-Diamino-diphenylmethan
4,4'-Diamino-diphenylmethan
N,N,N,N'-Tetramethyl-4,4'-diamino-diphenylmethan
1,2-Di-[(2-methyl-phenyl)-amino]-ethan
1,2-Di-(phenylamino)-propan
(o-Tolyl)-biguanid
Di-[4-(1',3'-dimethyl-butyl)-phenyl]amin
tert-octyliertes N-Phenyl-1-naphthylamin.

Beispiele für Metallpassivatoren sind

für Kupfer, z. B. : Benztriazol, Tetrahydrobenztriazol, 2-Mercaptobenzthiazol, 2,5-Dimercaptothiadiazol, Salicyliden-propylendiamin, Salze von Salicylaminoguanidin.

Beispiele für Rost-Inhibitoren sind

a) Organische Säuren, ihre Ester, Metallsalze und Anhydride, z. B. : N-Oleoyl-sarcosin, Sorbitanmono-oleat, Blei-naphthenat, Dodecenylbernsteinsäure-anhydrid, Alkenylbernsteinsäure-Halbester, 4-Nonylphenoxy-essigsäure.

b) Stickstoffhaltige Verbindungen, z. B. :

I. Primäre, sekundäre oder tertiäre aliphatische oder cycloaliphatische Amine und Amin-Salze von organischen und anorganischen Säuren, z. B. öllösliche Alkylammoniumcarboxylate.

II. Heterocyclische Verbindungen z. B. : Substituierte Imidazoline und Oxazoline.

c) Phosphorhaltige Verbindungen, z. B. : Aminsalze von Phosphorsäurepartialestern.

d) Schwefelhaltige Verbindungen, z. B. : Barium-dinonylnaphthalin-sulfonate, Calciumpetroleumsulfonate.

Beispiele für Viskositätsindex-Verbesserer sind z. B.

Polymethacrylate, Vinylpyrrolidon/Methacrylat-Copolymere, Polybutene, Olefin-Copolymere, Styrol/Acrylat-Copolymere.

Beispiele für Stockpunkterniedriger sind. z. B.

Polymethacrylat, alkylierte Naphthalinderivate.

Beispiele für Dispergiermittel/Tenside sind z. B. :

Polybutenylbernsteinsäure-imide, Polybutenylphosphonsäurederivate, basische Magnesium-, Calcium-, und Bariumsulfonate und -phenolate.

Beispiele für Hochdruck- und/oder verschleissmindernde Additive sind z. B. :

Schwefel und/oder Phosphor und/oder Halogen enthaltende Verbindungen, wie geschwefelte pflanzliche Oele, Zinkdialkyldithiophosphate, Tritolyl-phosphat, chlorierte Paraffine, Alkyl- und Aryldisulfide.

Beispiel 1

169,2 g Diphenylamin und 33,8 g aktive Tonerde (Fulcat® 22B von Laporte Industries) werden in ein mit Rührer und Temperaturfühler versehenes Reaktionsgefäss eingefüllt und auf 165 °C erhitzt. Sobald das Gemisch genügend leichtflüssig ist wird gerührt. Danach werden 196.4 g Diisobutylen nach und nach zudosiert, so dass die Temperatur des Reaktionsgemisches nicht unter 165 °C absinkt. Die Zugabe dauert 5 Stunden bis zur Beendigung der Reaktion. Rückfluss beginnt sogleich nach Reaktionsbeginn. Heizen und Rühren wird bei 165 °C weitergeführt unter häufiger Probenahme bis die Gas/Flüssigchromatographische Analyse einen Gehalt an 4,4'-Di-tert.-octyldiphenylamin von unter 25 Gew.-% ergibt (ohne Katalysator).

Die Reaktionsmasse wird auf 60 °C gekühlt und der Katalysator durch Vakuumfiltration entfernt. Das Filtrat wird in eine Destillations-apparatur übergeführt und unter Heizen und Rühren wird der Druck auf 2 600 Pa reduziert.

Während der Destillation lässt man die Aussentemperatur langsam auf 165 °C steigen und hält sie über 2 Stunden konstant auf dieser Temperatur während die Destillation zum Stillstand kommt. Es werden 300 g einer viskosen, dunklen Flüssigkeit vom Flammpunkt von 210 °C erhalten.

## Beispiele 2 bis 6

Die Reaktionsbedingungen, wie in Beispiel 1 beschrieben, werden in Bezug auf die Parameter Temperaturbereich und Molverhältnis von Diphenylamin (DPA) zu Diisobutylen (DIB) variiert.

Die Resultate sind in Tabelle I widergegeben.

### Tabelle I

| Bei-spiel | Temperatur-bereich (°C) | Molver-hältnis DPA/DIB | DIB Zugabe-Zeit (Std.) | Gesamt-Reaktions-Zeit (Std.) | Aggregatszustand d.Produkts |
|---|---|---|---|---|---|
| 2 | 160–170 | 1:2.5 | 5 | 29 | flüssig |
| 3 | 169–170 | 1:2.5 | 19 | 24 | flüssig |
| 4 | 168–170 | 1:2.1 | 30 | 30 | flüssig |
| 5 | 165–170 | 1:2.0 | 17 | 30 | flüssig |
| 6 | 160–170 | 1:2.2 | 6.25 | – | flüssig |

## Patentansprüche

1. Verfahren zur Herstellung einer flüssigen Antioxidans-Zusammensetzung durch Reaktion von Diphenylamin mit Diisobutylen, dadurch gekennzeichnet, dass die Reaktion von Diphenylamin mit einem Ueberschuss an Diisobutylen in Anwesenheit eines aktiven Tonerde-Katalysators durchgeführt wird, dass die Konzentration an Diisobutylen über die Reaktionsdauer im Wesentlichen konstant gehalten wird, dass die Reaktionstemperatur mindestens 160 °C beträgt, dass die Reaktion solange durchgeführt wird bis der Gehalt an 4,4'-Di-tert.-octyldiphenylamin, bezogen auf die Reaktionsmasse ohne Katalysator, unter 30 Gew.-%, und der Gehalt an Diphenylamin unter 10 Gew.-%, liegen, dass der Katalysator und nicht umgesetztes Diisobutylen entfernt werden und dass das entstehende flüssige Produkt, enthaltend mindestens die Komponenten 1) tert.-Butyldiphenylamine, 2) tert.-Octyldiphenylamine und 3) höher alkylierte Diphenylamine, isoliert wird.

2. Verfahren nach Anspruch 1, worin der Gehalt an 4,4'-Di-tert.-octyldiphenylamin unter 25 Gew.-% und der Gehalt an Diphenylamin unter 5 Gew.-% liegt.

3. Verfahren gemäss Anspruch 1, worin die Temperatur, bei der das Verfahren durchgeführt wird, im Bereich von 160 °C bis 250 °C liegt.

4. Verfahren gemäss Anspruch 3, worin die Temperatur, bei der das Verfahren durchgeführt wird, im Bereich von 160 °C bis 190 °C liegt.

5. Verfahren gemäss Anspruch 1, worin das Molverhältnis von Diphenylamin zu Diisobutylen im Bereich von 1 : 1.11 bis 1 : 2.5 liegt.

6. Ein Verfahren gemäss Anspruch 5, worin das Molverhältnis von Diphenylamin zu Diisobutylen im Bereich von 1 : 1.3 bis 1 : 1.75 liegt.

7. Verfahren gemäss Anspruch 1, worin der verwendete aktive-Tonerde-Katalysator einen freien Feuchtigkeitsgehalt von unter 10 Gew.-% aufweist.

8. Verfahren gemäss Anspruch 7, worin der verwendete aktive-Tonerde-Katalysator einen freien Feuchtigkeitsgehalt von unter 5 Gew.-% aufweist.

9. Verfahren gemäss Anspruch 1, worin der verwendete Katalysator eine mit Schwefelsäure aktivierte Tonerde ist.

7

10. Verfahren gemäss Anspruch 1, worin die Menge des eingesetzten Katalysators 5-25 Gew.-% bezogen auf das Gewicht der Diphenylamin-Reaktionskomponente beträgt.

11. Verfahren gemäss Anspruch 10, worin die Menge des eingesetzten Katalysators 5 bis 15 Gew.-% bezogen auf das Gewicht der Diphenylamin-Reaktionskomponente beträgt.

12. Verfahren gemäss Anspruch 1, worin die Reaktionsdauer so gross ist, dass der Gehalt an 4,4'-Di-tert.-octyldiphenylamin bezogen auf die Reaktionsmasse ohne Katalysator unter 25 Gew.-% und der Gehalt an Diphenylamin unter 5 Gew.-% liegen.

13. Flüssige Antioxidans-Zusammensetzung hergestellt durch die Reaktion von Diphenylamin mit einem molaren Ueberschuss an Diisobutylen in Anwesenheit eines aktiven Tonerde-Katalysators, wobei die Konzentration an Diisobutylen über die Reaktionsdauer im Wesentlichen konstant gehalten wird, bei einer Reaktionstemperatur von mindestens 160 °C und einer Reaktionsdauer solange bis der Gehalt an 4,4'-Di-tert.-octyldiphenylamin bezogen auf die Reaktionsmasse ohne Katalysator unter 30 Gew.-% und der Gehalt an Diphenylamin unter 10 Gew.-% liegen und wobei der Katalysator und nicht umgesetztes Diisobutylen entfernt werden und das enstehende flüssige Produkt, enthaltend mindenstens die Komponenten 1) tert-Butyldiphenylamine, 2) tert-Octyldiphenylamine und 3) höher alkylierte Diphenylamine, isoliert wird.

14. Zusammensetzung enthaltend ein dem oxidativen Abbau unterliegendes organisches Material und als Stabilisator einen stabilisierenden Anteil eines flüssigen Antioxidans gemäss Anspruch 13.

15. Zusammensetzung gemäss Anspruch 14 worin das organische Material ein Schmiermittel oder eine funktionelle Flüssigkeit ist.

16. Zusammensetzung gemäss Anspruch 14, wobei der Anteil des flüssigen Antioxidans bezogen auf das Gewicht des organischen Materials im Bereich von 0.05 Gew.-% bis 5 Gew.-% liegt.

17. Zusammensetzung gemäss Anspruch 15 worin das Schmiermittel ein oder mehrere weitere Antioxidantien, Metallpassivatoren, Rostinhibitoren, Viscositätsindex-Verbesserer, Stockpunkterniedriger, Dispergiermittel, Detergentien und Antiverschleiss-Additive enthält.

**Claims**

1. Process for the production of a liquid antioxidant composition by reaction of diphenylamine with diisobutylene comprising reacting diphenylamine with an excess of diisobutylene in the presence of an activated clay catalyst, whilst ensuring that the concentration of diisobutylene remains substantially constant throughout the reaction period, at a reaction temperature of at least 160 °C, the reaction being effected for such a period that the content of 4,4'-di-tert.-octyldiphenylamine, based on the reaction mass, excluding catalyst, is below 30 % by weight and the content of diphenylamine is below 10 % by weight ; removing catalyst and unreacted diisobutylene, and isolating the resulting liquid product, containing at least the components 1) tert-butyldiphenylamines, 2) tert-octyldiphenylamines and 3) higher alkylated diphenylamines.

2. Process according to claim 1, wherein the content of 4,4'-di-tert-octyldiphenylamine is below 25 % by weight and the content of diphenylamine is below 5 % by weight.

3. Process according to claim 1, wherein the temperature at which the process is effected is within the range from 160 °C to 250 °C.

4. Process according to claim 3, wherein the temperature at which the process is effected is within the range from 160 °C to 190 °C.

5. Process according to claim 1, wherein the molar ratio of diphenylamine to diisobutylene is within the range of from 1 : 1.11 to 1 : 2.5.

6. Process according to claim 5, wherein the molar ratio of diphenylamine to diisobutylene is within the range from 1 : 1.3 to 1 : 1.75.

7. Process according to claim 1, wherein the activated clay catalyst used has a free moisture content below 10 % by weight.

8. Process according to claim 7, wherein the activated clay catalyst used has a free moisture content below 5 % by weight.

9. Process according to claim 1, wherein the catalyst used is a sulfuric acid-activated clay.

10. Process according to claim 1, wherein the amount of catalyst used is from 5 to 25 % by weight, based on the weight of diphenylamine reactant.

11. Process according to claim 10, wherein the amount of catalyst used is from 5 to 15 % by weight, based on the weight of diphenylamine reactant.

12. Process according to claim 1, wherein the reaction is effected for such a reaction period that the content of 4,4'-di-tert-octyldiphenylamine, based on the reaction mass, excluding catalyst, is below 25 % by weight, and the content of diphenylamine is below 5 % by weight.

13. Liquid antioxidant composition produced by reacting diphenylamine with a molar excess of diisobutylene in the presence of an activated clay catalyst, whilst ensuring that the concentration of diisobutylene remains substantially constant throughout the reaction period, at a reaction temperature of at least 160 °C, the reaction being effected for such a period that the content of 4,4'-di-tert-octyldiphenylamine, based on the reaction mass, excluding catalyst, is below 30 % by weight and the content of diphenylamine is below 10 % by weight ; removing catalyst and unreacted diisobutylene, and isolating the resulting liquid product, containing at least the components 1) tert-butyldiphenylamines, 2) tert-octyldiphenylamines and 3) higher alkylated diphenylamines.

14. Composition containing an organic material susceptible to oxidative degradation and, as stabilizer, a stabilizing amount of a liquid antioxidant according to claim 13.

15. Composition according to claim 14, wherein the organic material is a lubricant or functional fluid.

16. Composition according to claim 14, wherein the proportion of liquid antioxidant is within the range of from 0.05 % to 5 % by weight, based on the weight of the organic material.

17. Composition according to claim 15, wherein the lubricant also contains one or more further antioxidants, metal passivators, rust inhibitors, viscosity index improvers, pour-point depressors, dispersants, surfactants or anti-wear additives.


**Revendications**

1. Procédé pour préparer une composition antioxydante liquide par réaction de la diphénylamine avec le di-isobutène, procédé caractérisé en ce qu'on effectue la réaction de la diphénylamine avec un excès de di-isobutène en présence d'un catalyseur actif à base d'alumine, on maintient pratiquement constante la concentration en di-isobutène pendant toute la durée de la réaction, la température réactionnelle est d'au moins 160 °C, on poursuit la réaction jusqu'à ce que la teneur en di-tert-octyl-4,4' diphénylamine, par rapport au mélange réactionnel sans catalyseur, soit inférieure à 30 % en poids et la teneur en diphénylamine inférieure à 10 % en poids, on élimine le catalyseur et le di-isobutène qui n'a pas réagi, et on isole le produit liquide formé, qui contient au moins les composantes suivantes : 1) des tert-butyl-diphénylamines, 2) des tert-octyl-diphénylamines et 3) des diphénylamines à alkylation supérieure.

2. Procédé selon la revendication 1 dans lequel la teneur en di-tert-octyl-4,4' diphénylamine est inférieure à 25 % en poids et la teneur en diphénylamine inférieure à 5 % en poids.

3. Procédé selon la revendication 1 dans lequel la température à laquelle le procédé est exécuté est comprise entre 160 et 250 °C.

4. Procédé selon la revendication 3 dans lequel la température à laquelle le procédé est exécuté est comprise entre 160 et 190 °C.

5. Procédé selon la revendication 1 dans lequel le rapport molaire de la diphénylamine au di-isobutène est compris entre 1 : 1,11 et 1 : 2,5.

6. Procédé selon la revendication 5 dans lequel le rapport molaire de la diphénylamine au di-isobutène est compris entre 1 : 1,3 et 1 : 1,75.

7. Procédé selon la revendication 1 dans lequel le catalyseur actif à base d'alumine dont on se sert a une teneur en humidité libre inférieure à 10 % en poids.

8. Procédé selon la revendication 7 dans lequel le catalyseur actif à base d'alumine dont on se sert a une teneur en humidité libre inférieure à 5 % en poids.

9. Procédé selon la revendication 1 dans lequel le catalyseur utilisé est une alumine activée par de l'acide sulfurique.

10. Procédé selon la revendication 1 dans lequel la quantité du catalyseur mis en jeu est de 5 à 25 % en poids par rapport au poids de la diphénylamine, qui est une composante réactionnelle.

11. Procédé selon la revendication 10 dans lequel la quantité du catalyseur mis en jeu est de 5 à 15 % en poids par rapport au poids de la diphénylamine, qui est une composante réactionnelle.

12. Procédé selon la revendication 1 dans lequel la durée de la réaction est suffisamment longue pour que la teneur en di-tert-octyl-4,4' diphénylamine, par raport au mélange réactionnel sans catalyseur, soit inférieure à 25 % en poids et la teneur en diphénylamine inférieure à 5 % en poids.

13. Composition antioxydante liquide préparée par réaction de la diphénylamine avec un excès molaire de di-isobutène en présence d'un catalyseur actif à base d'alumine, la concentration en di-isobutène étant maintenue pratiquement constante pendant toute la durée de la réaction, la réaction étant effectuée à au moins 160 °C et poursuivie jusqu'à ce que la teneur en di-tert-octyl-4,4' diphénylamine, par rapport au mélange réactionnel sans catalyseur, soit inférieure à 30 % en poids et la teneur en diphénylamine inférieure à 10 % en poids, le catalyseur et le di-iso-butène qui n'a pas réagi étant éliminés et le produit liquide formé, qui contient au moins les composantes suivantes : 1) des tert-butyl-diphénylamines, 2) des tert-octyl-diphénylamines et 3) des diphénylamines à alkylation supérieure, étant isolé.

14. Composition qui contient une matière organique sensible à la dégradation par oxydation et, comme stabilisant, une proportion stabilisante d'un antioxydant liquide selon la revendication 13.

15. Procédé selon la revendication 14 dans laquelle la matière organique est un lubrifiant ou un liquide fonctionnel.

16. Composition selon la revendication 14 dans laquelle la proportion de l'antioxydant liquide, par rapport au poids de la matière organique, est comprise entre 0,05 % en poids et 5 % en poids.

17. Composition selon la revendication 15 dans laquelle le lubrifiant contient un ou plusieurs antioxydants supplémentaires, des passivants de métaux, des inhibiteurs de corrosion, des améliorateurs d'indice de viscosité, des abaisseurs de point de congélation, des dispersants, des détergents et des additifs anti-usure.